# EUROPEAN PATENT SPECIFICATION

(11) **EP 1 272 195 B1**
(45) Date of publication and mention of the grant of the patent: **12.10.2005**
(21) Application number: 01917289.9
(22) Date of filing: 02.04.2001
(51) Int. Cl.: A61K 31/565, A61P 35/00

(54) **USE OF FULVESTRANT IN THE TREATMENT OF RESISTANT BREAST CANCER**
VERWENDUNG VON FULVESTRANT IN DER BEHANDLUNG VON RESISTENTEM BRUSTKREBS
UTILISATION DE FULVESTRANT DANS LE TRAITEMENT DU CANCER RESISTANT DU SEIN

(30) Priority: 05.04.2000 GB 0008172
(43) Date of publication of application: 08.01.2003
(60) Divisional application: 05007561.3
(73) Proprietor: AstraZeneca AB, 151 85 Södertälje (SE)
(72) Inventor: THURLIMANN, Beat, CH-9007 St. Gallen (CH)
(74) Representative: Giles, Allen Frank
(86) International application number: PCT/GB2001/001500
(87) International publication number: WO 2001/074366

(56) References cited:
- BRODIE A ET AL: "Aromatase inhibitors and their antitumor effects in model systems." DGR, (1999). VOL. 6, NO. 2, PP. 205-10. JOURNAL CODE: DGR. ISSN: 1351-0088., XP001007660 Department of Pharmacology and Experimental Therapeutics, and Greenebaum Cancer Center, School of Medicine, University of Maryland, Baltimore 21201, USA.
- ENGLAND GALE M ET AL: "Pure antiestrogens as a new therapy for breast cancer." ONCOLOGY RESEARCH, vol. 9, no. 8, 1997, pages 397-402, XP001007972 ISSN: 0965-0407
- HOWELL A ET AL: "Clinical studies with the specific 'pure' antioestrogen ICI 182780." BREAST, vol. 5, no. 3, 1996, pages 192-195, XP001007973 ISSN: 0960-9776
- PARISOT JOHN P ET AL: "Induction of insulin-like growth factor binding protein expression by ICI 182,780 in a tamoxifen-resistant human breast cancer cell line." BREAST CANCER RESEARCH AND TREATMENT, vol. 55, no. 3, June 1999 (1999-06), pages 231-242, XP001007971 ISSN: 0167-6806
- HU X F ET AL: "Circumvention of tamoxifen resistance by the pure anti-estrogen ICI 182,780." INTERNATIONAL JOURNAL OF CANCER, vol. 55, no. 5, 1993, pages 873-876, XP001007968 ISSN: 0020-7136

## Description

The invention relates to the use of fulvestrant in the treatment of breast cancer in patients who have previously been treated with tamoxifen and an aromatase inhibitor.

Breast Cancer is the most common malignancy to affect women and represents 18% of all female cancers (McPherson *et al* 1994). Worldwide, the incidence of the disease is increasing, and each year over a quarter of a million deaths are caused by breast cancer. Over half a million new cases are diagnosed each year, of which about half occur in North America and Western Europe.

It has long been recognised that many breast cancers are hormone dependent and that hormonal manipulation can affect the progress of the disease. The first responses of metastatic breast cancer to hormone manipulation by bilateral oophorectomy were reported in 1896 (Beatson 1896).

Estrogens, in particular, act as endocrine growth factors for at least one-third of breast cancers, and depriving the tumour of this stimulus is a recognised therapy for advanced disease (Muss 1992). Note "estrogen" and "oestrogen" are mere alternative spellings.

In premenopausal women, this is achieved by the ablation of ovarian function through surgical, radiotherapeutic, or medical means and, in postmenopausal women, by the use of aromatase inhibitors.

An alternative approach to estrogen withdrawal is to antagonise estrogen with antiestrogens. These are drugs that bind to and compete for estrogen receptors (ER) present in estrogen-responsive tissue. Conventional nonsteroidal antiestrogens, such as tamoxifen, compete efficiently for ER binding but their effectiveness is often limited by the partial agonism they display, which results in an incomplete blockade of estrogen-mediated activity (Furr and Jordan 1984, May and Westley 1987).

Sequential hormonal treatment represents an established approach for treatment of hormone-sensitive advanced breast cancer. In postmenopausal women with breast cancer whose disease has progressed following treatment with tamoxifen, which is a SERM, the 3^{rd} generation non-steroidal aromatase inhibitors, such as anastrozole and letrozole, are the treatment of choice (Buzdar 1999). Of particular interest remains the question of treatment after failure of 3^{rd} generation non-steroidal aromatase inhibitors.

A specific or "pure" antiestrogen with high affinity for ER and without any agonist effects, may have advantages over conventional nonsteroidal antiestrogens in the treatment of estrogen-dependent disease. The search for such a drug revealed a number of compounds with the appropriate effect (Wakeling and Bowler 1987, 1988, Bowler *et al* 1989) of which fulvestrant (Faslodex™, ZD9238, ICI 182,780) was selected for further development.

Fulvestrant is the first of a new class of potent pure antiestrogens and is completely free of the partial agonist, estrogen-like activity, associated with currently available antiestrogens like tamoxifen. Fulvestrant has already demonstrated efficacy in a phase II trial in women whose breast cancer has progressed following tamoxifen therapy (Howell *et al* 1995).

Fulvestrant is a pure antiestrogen with a novel mechanism of action, described as an Estrogen Receptor Downregulator (E.R.D.), with clear evidence of anti-tumour activity in advanced breast cancer (Howell *et al* 1995, 1996).

Fulvestrant, (7-alpha-[9-(4,4,5,5,5-pentafluoropentylsulphinyl)nonyl]-estra-1,3,5(10)-triene-3, 17β diol), is a new compound that has been shown to have significant pure estrogen antagonist activity with no agonist effect (Wakeling *et al* 1991). The relative binding affinity of fulvestrant for the estrogen receptor (ER) is 0.89, compared with an oestradiol equivalent of 1.0. Fulvestrant has a marked inhibitory effect on the growth of MCF-7 human breast cancer cells (IC₅₀ 0.29 nM) and produced an 80% reduction in MCF-7 cell numbers under conditions where tamoxifen produced a maximum 50% inhibition (Wakeling *et al* 1991).

Studies have shown that tamoxifen-resistant MCF-7 tumours, which grow out after long-term treatment with tamoxifen, remain sensitive to fulvestrant treatment (Osbome *et al* 1994). The same investigators also showed that treatment with fulvestrant suppressed the growth of established MCF-7 tumours for twice as long as treatment with tam oxifen, and tumourgenesis was delayed to a greater extent in fulvestrant treated mice than in tamoxifen-treated mice. (Osborne *et al* 1995).

We have surprisingly found that patients who have previously been treated with an aromatase inhibitor, which patients have also have previously been treated with tamoxifen, and in which they have failed previous treatment(s), have breast cancer which is sensitive to further treatment by fulvestrant.

A feature of the invention is the use of fulvestrant in the preparation of a medicament for the treatment of a patient with breast cancer who previously has been treated with an aromatase inhibitor and tamoxifen (Nolvadex™), and have failed with such previous treatment.

Preferably the patient has not been prior treated for breast cancer with more than 2 different hormonal agents.

Preferably the patient is human, especially female.

Preferably the aromatase inhibitor is anastrozole (Arimidex™), letrozole (Femara™) or aminoglutethimide (Orimeten™). More preferably the aromatase inhibitor is anastrozole.

By the use of the term "failed" we mean that growth of the breast cancer is no longer arrested by treatment with an aromatase inhibitor, or tamoxifen, or both an aromatase inhibitor and tamoxifen together.

Fulvestrant may be administered by any suitable route of administration. A preferred route of administration is by intra-muscular injection, preferably in a castor oil based long acting formulation, preferably at the dose of at least 200mg, preferably at intervals of at least a month. Especially preferred is 200 - 300mg fulvestrant given intramuscularly in a castor oil based formulation, preferably at intervals of at least one month. Most preferred is about 250mg of fulvestrant preferably given at approximately monthly intervals. Preferably doses should be administered so as to achieve blood serum levels of fulvestrant of at least 2.5 ng/ml, more preferably from 5 to 20 ngml⁻¹.

Preferably fulvestrant is administered as a medicament which is a pharmaceutical formulation adapted for intra-muscular injection comprising fulvestrant, 30% or less weight of a pharmaceutically-acceptable alcohol per volume of formulation, at least 1% weight of a a pharmaceutically-acceptable alcohol per volume of formulation, at least 1% weight of a pharmaceutically-acceptable non-aqueous ester solvent miscible in a ricinoleate vehicle per volume of formulation and a sufficient amount of a ricinoleate vehicle so as to prepare a formulation of at least 45mgml⁻¹ of fulvestrant. By the use of the term ricinoleate vehicle we mean an oil which has as a proportion (at least 20%, 30%, 40%, 50%, 60%, 70%, 80%, 90% or 95% w/v) of its composition as triglycerides of ricinoleic acid. The ricinoleate vehicle may be a synthetic oil or conveniently is castor oil, ideally of pharmacopoeial standards, as described above.

For the avoidance of any doubt when using the term % weight per volume of formulation for the constituents of the formulation we mean that within a unit volume of the formulation a certain percentage of the constituent by weight will be present, for example a 1% weight per volume formulation will contain within a 100ml volume of formulation 1g of the constituent. By way of further illustration

| % of x by weight per volume of formulation | weight of x in 1ml of formulation |
|---|---|
| 30% | 300mg |
| 20% | 200mg |
| 10% | 100mg |
| 5% | 50mg |
| 1% | 10mg |

Preferably the pharmaceutical formulation contains 25% w/v or less of a pharmaceutically-acceptable alcohol, more preferably the pharmaceutical formulation contains 20% w/v or less of a pharmaceutically-acceptable alcohol.

Preferably the pharmaceutical formulation contains 60% w/v or less of a pharmaceutically-acceptable non-aqueous ester solvent. More preferably the pharmaceutical formulation contains 50%w/v or less of a pharmaceutically-acceptable non-aqueous ester solvent . More preferably the pharmaceutical formulation contains 45% w/v or less of a pharmaceutically-acceptable non-aqueous ester solvent. More preferably the pharmaceutical formulation contains 40% w/v or less of a pharmaceutically-acceptable non-aqueous ester solvent. More preferably the pharmaceutical formulation contains 35% w/v or less of a pharmaceutically-acceptable non-aqueous ester solvent. More preferably the pharmaceutical formulation contains 30% w/v or less of a pharmaceutically-acceptable non-aqueous ester solvent. More preferably the pharmaceutical formulation contains 25% w/v or less of a pharmaceutically-acceptable non-aqueous ester solvent.

Preferably the pharmaceutical formulation has the pharmaceutically-acceptable alcohol as a mixture of ethanol and benzyl alcohol.

Preferably the pharmaceutical formulation has the pharmaceutically-acceptable non-aqueous ester solvent selected from benzyl benzoate, ethyl oleate, isopropyl myristate, isopropyl palmitate or a mixture of any thereof. More preferably the pharmaceutically-acceptable non-aqueous ester solvent is benzyl benzoate.

A preferred pharmaceutical formulation has the concentration of fulvestrant of at least 45mgml⁻¹. A preferred pharmaceutical formulation is a formulation wherein the total amount of fulvestrant in the formulation is 250mg, or more, and the total volume of the formulation is 6ml, or less.

An especially preferred pharmaceutical formulation is a formulation wherein the pharmaceutically-acceptable alcohol is a mixture of 10% weight of ethanol per volume of formulation, 10% weight of benzyl alcohol per volume of formulation and 15% weight of benzyl benzoate per volume of formulation and the ricinoleate vehicle is castor oil.

### REFERENCES

Beatson GT, on the treatment of inoperable cases of carcinoma of the mamma: Suggestions for a new method of treatment with illustrative cases. Lancet 1896; 2: 104-107.
Bowler J, Lilley TJ, Pittam JD, Wakeling AE, Novel steroidal pure antioestrogens.
Steroids 1989; 54: 71-99.
Buzdar A, Role of aromatase inhibitors in advanced breast cancer. Endocrine-Related Cancer 1999; 6 (2): 219-225.
Furr BJA, Jordan VC, The pharmacology and clinical uses of tamoxifen. Pharmaceutical Therapy 1984; 25:127-205
Howell A, DeFriend D, Robertson J, Blarney R, Walton P, Response to a specific antioestrogen (ICI 182,780) in tamoxifen-resistant breast cancer. Lancet 1995; 345: 29-30.
Howell A, DeFriend DJ, Robertson JFR, Blamey R *et al*, Pharmacokinetics, pharmacological and antitumour effects of the specific antioestrogen ICI 182,780 in women with advanced breast cancer. Br J Cancer 1996; 74: 300-308.
Jacobs Th W, Comparison of fluorescence in situ hybridization and immunohistochemistry for the evaluation of *HER-2*/*neu* in breast cancer. Journal of Clinical Oncology 1999, 17: 1974-1982
May FEB, Westley BR, Effects of tamoxifen and 4'-hydroxytamoxifen on the pNR-1 and pNR-2 estrogen-regulated RNAs in human breast cancer cells. Journal Biological Chemistry 1987; 262:15894-9.
McPershon K, Steel CM, Dixon JM, Breast cancer - epidemiology, risk factors and genetics. British Medical Journal 1994; 309: 1003-6.
Muss MD, Endocrine therapy for advanced breast cancer. Breast Cancer Research and Treatment 1992; 21:15-26.
Osborne CK, Jarman M, McCague R, Coronado EB, Hilsenbeck SG, Wakeling AE, The importance of tamoxifen metabolism in tamoxifen-stimulated breast tumour growth. Cancer Chem Pharmacol 1994; 34: 89-95.
Osborne CK, Coronado-Heinsohn EB, McCue BL, Wakeling AE, McLelland RA, Manning DL, Nicholson RI, Comparison of the effects of a pure steroidal antioestrogen with those of tamoxifen in a model of human breast cancer. J Nat Cancer Inst 1995; 87: 746-750.
Simon R, Optimal two-stage designs for Phase II clinical trials. Controlled Clinical Trials 10:1-10, 1989.
Wakeling AE, Bowler J, Steroidal pure antioestrogens. J. Endocrinol 1987; 112: R7-R10.
Wakeling AE, Bowler J, Biology and mode of action of pure antioestrogens.J Steroid. Biochem 1988; 30: 141-148.
Wakeling AE, Dukes M, Bowler J, A Potent specific pure antioestrogen with clinical potential. Cancer Res 1991; 51: 3867-3873.

### TABLE OF ABBREVIATIONS

| | |
|---|---|
| AE | adverse event |
| ALP | alkaline phesphatase |
| ALT | alanine aminotransferase |
| AST | aspartate transaminase |
| BP | blood pressure |
| CR | complete response |
| CRF | case record form |
| CT | computed tomography |
| ER | estrogen receptor |
| ERD | estrogen receptor downregulator |
| FISH | fluorescence in situ hybridisation |
| HR | heart rate |
| INR | International Normalised Ratio (clotting time) |
| LH-RH | luteinising hormone releasing hormones |
| LP | local pathodogy |
| MRI | magnetic resonance imaging |
| PCR | polymerase chain reaction |
| PgR | progesterone receptor |
| PR | partial response |
| SD | stable disease |
| SERM | selective estrogen receptor modulator |
| ULRR | upper limit of the reference range |

The invention is illustrated by the following non-limiting example which is a summary of a clinical protocol looking at the effects of fulvestrant in breast cancer.

### Example 1 - Clinical Trial Protocol

- TITLE:: An open, multicenter phase II trial evaluating the antitumour efficacy of Faslodex® (fulvestrant) in postmenopausal women with advanced breast cancer failing non-steroidal aromatase inhibitors
- OBJECTIVES:: Primary: Objective response rate (= CR + PR) of fulvestrant treatment Secondary: Duration of clinical benefit (= CR + PR + SD ≥ 24 weeks), time to progression, duration of response, time to treatment failure and safety and tolerability of fulvestarnt treatment Objective response rate according to Her-2/*neu* status
- TRIAL DESIGN:: Open, multicentre, non-comparative phase II trial. Patients enrolled according to three levels of stratification.
Stratum A: patients responsive to anastrozole or letrozole or aminoglutethimide treatment given for advanced disease
Stratum B: patients resistant to anastrozole or letrozole or aminoglutethimide treatment given for advanced disease
Stratum C: eligible patients for whom strata A and B are not applicable, i.e. received anastrozole, letrozole or aminoglutethimide as adjuvant therapy.
- TYPE ANDNUMBER OFPATIENTS:: Patients will be postmenopausal women with advanced breast cancer who have progressed following anastrozole or letrozole or aminoglutethimide treatment.
- TRIAL TREATMENT:: Fulvestrant as a 250 mg intramuscular (im) injection once every month
- DURATION OF TREATMENT:: Patients may continue treatment until objective evidence of breast cancer progression.
- PRIMARY ENDPOINT: Objective response rate
- SECONDARY ENDPOINTS: Duration of clinical benefit, time to progression, duration of response, time to treatment failure, tolerability and safety objective response rate according to HER-2/*neu* status
- ANALYSIS: When all patients have been followed-up for at least 24 weeks, the final analysis will be performed to assess:
objective tumour response (primary measure)
duration of clinical benefit
time to progression
duration of response
time to treatment failure
tolerability and safety

Objective response rate according to HER-2/*neu* status will also be assessed

### TRIAL PLAN

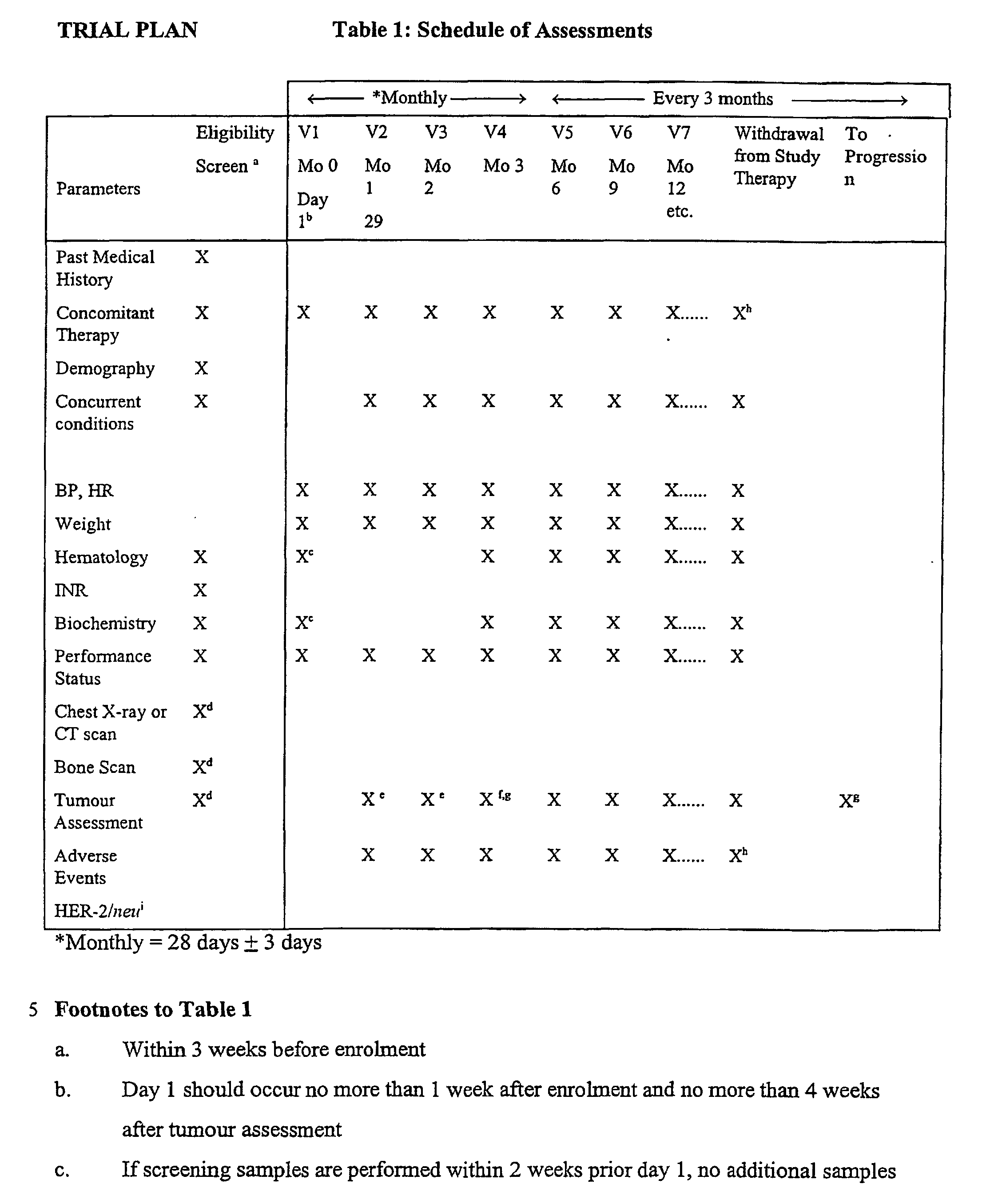

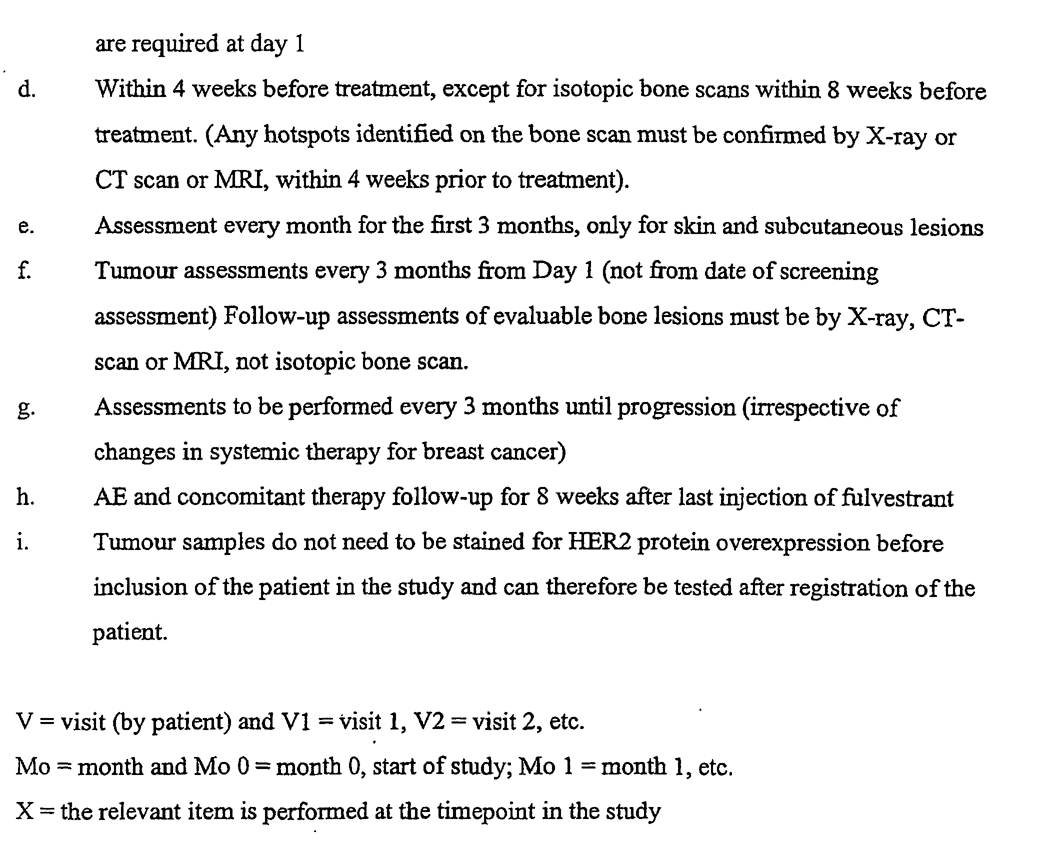

### OBJECTIVES

### Primary:

The primary objective of this trial is to evaluate the antitumour efficacy of Faslodex (fulvestrant) in terms of objective response rate in postmenopausal women with advanced breast cancer failing anastrozole or letrozole or aminoglutethimide.

### Secondary:

The secondary objectives are:
- Duration of clinical benefit, time to progression, duration of response, time to treatment failure
- Tolerability (local and systemic) and safety of fulvestrant
- Objective response rate according to HER-2/*neu* status

### TRIAL DESIGN

### 1.1 Design

An open, multicentre, non-comparative phase II trial conducted in postmenopausal females with advanced breast cancer.

Patients who meet the eligibility criteria receive fulvestrant 250 mg (5.0 ml) im injection every month (28 days ± 3 days). The trial involves three levels of stratification:
- **Stratum A:** anastrozole or letrozole or aminoglutethimide responsive patients defined as patients who progressed while on anastrozole or letrozole or aminoglutethimide treatment given for advanced disease after initial objective response or disease stabilisation of at least 24 weeks
- **Stratum B:** anastrozole or letrozole or aminoglutethimide resistant patients defined as patients who did not respond to anastrozole or letrozole or aminoglutethimide given for advanced disease or showed disease stabilisation lasting less than 24 weeks
- **Stratum C:** eligible patients for whom strata A and B are not applicable, i.e. received anastrozole or letrozole or aminoglutethimide as adjuvant therapy

Patients receive fulvestrant until there is objective evidence of disease progression or other events necessitating treatment withdrawal. At that time, trial treatment will be stopped and a new treatment option will be discussed with the patient. For patients who withdraw from treatment for reasons other than disease progression efficacy assessments should continue to be performed as protocolled until disease progression is observed.

The final analysis is performed when all patients have been followed-up for at least 24 weeks to assess:
objective response rate (primary measure)
duration of clinical benefit
time to progression
duration of response
time to treatment failure
tolerability and safety

Objective response rate according to Her-2/*neu* status will also be assessed.

Patients will attend for full assessment until withdrawal from trial therapy. Patients who have not progressed at withdrawal from trial therapy will continue to be assessed for tumour status until progression of disease occurs. All patients should be followed up until there is objective progression of disease, irrespective of changes in systemic therapy for breast cancer. Date of change and type of alternative cancer therapy will be recorded.

### 2 PATIENT SELECTION

### 2.1 Inclusion criteria

For a patient to be eligible for entry into the trial all of the following must be met:
a) Histological/cytological confirmation of breast cancer
b) Objective evidence of progression of disease under treatment with a non-steroidal aromatase inhibitor. Non-steroidal aromatase inhibitor is defined as anastrozole or letrozole or aminoglutethimide
c) Postmenopausal woman, defined as a woman fulfilling any of the following criteria:
   i) age ≥55 years
   ii) age ≥45 years with amenorrhea >12 months and an intact uterus
   iii) biochemical evidence of postmenopausal hormonal status, or
   iv) having undergone a bilateral oophorectomy
d) Indication for hormonal treatment for metastatic breast cancer following progression on anastrozole or letrozole or aminoglutethimide after at least 12 weeks treatment with anastrozole 1mg/d or letrozole 2.5mg/d or aminoglutethimide ≥ 500mg/d
e) Evidence of hormone sensitivity defined as:
   at least 12 months' adjuvant hormonal treatment before relapse,
      or
   tumour remission or stabilisation for at least 3 months resulting from hormone therapy before progression in advanced disease,
      or
   estrogen or progesterone receptor positive (ER+ve or PgR+ve). For this trial, ER+ve is defined as ≥ 10 fmol/mg cytosol protein binding to ER or ≥ 10% of the tumour cells stained for these receptors. PgR+ve is defined as ≥ 10 fmol/mg cytosol protein binding to PgR or ≥ 10% of the tumour cells stained for these receptors.
f) Presence of at least one measurable or evaluable lesion
g) Performance status of 0, 1 or 2
h) Life expectancy of more than 3 months
i) Written informed consent to participate in the trial

### 2.2 Exclusion criteria

Any one of the following is sufficient to exclude a patient from entering the trial:
a) Presence of life-threatening metastatic visceral disease defined as extensive hepatic involvement, or any degree of brain and/or leptomeningeal involvement (past or present), or symptomatic pulmonary lymphangitic spread. Patients with discrete pulmonary parenchymal metastases are eligible, provided their respiratory function is not compromised as a result of disease
b) Previous treatment with a progestin, oestrogens, androgens or fulvestrant for breast cancer
c) Prior treatment for breast cancer with more than 2 different hormonal agents (patients who have received tamoxifen or other anti-oestrogens as both adjuvant treatment and for advanced disease prior to anastrozole or letrozole or aminoglutethimide treatment are eligible). For this trial ovarian ablation in the forin of oophorectomy, ovarian irradiation, and LH-RH analogue therapy are not considered to be endocrine treatments.
d) Treatment with luteinising hormone releasing hormones (LH-RH) analogues ≤ 3 months prior to enrolment. Patients who have received prior LH-RH analogue therapy greater than 3 months prior to enrolment will be excluded if they have restarted menses or do not have castrate FSH levels within the postmenopausal range (as determined using ranges from the testing laboratory facility).
e) More than 1 chemotherapy for advanced disease before anastrozole or letrozole or aminoglutethimide therapy
f) Extensive radiotherapy within the last 4 weeks (i.e. ≥30% of bone marrow, e.g. whole of pelvis or half of spine)
g) Treatment with bisphosphonates within the last 8 weeks if evaluable bone lesions only
h) Current or previously active systemic malignancy within the past 3 years (other than breast cancer, or adequately treated in-situ carcinoma of the cervix uteri, or basal or squamous cell carcinoma of the skin);
i) Plan to receive or plan to continue to receive oestrogen replacement therapy
j) - platelets <100 x 10⁹/l; INR >1.6
   - total bilirubin >1.5 x the upper limit of the reference range (ULRR)
      - ALT or AST >2.5 x ULRR if no demonstrable liver metastases
   - or >5 x ULRR in presence of liver metastases;
k) Treatment with a non-approved or experimental drug within 4 weeks
l) Any evidence of severe or uncontrolled systemic disease (e.g. severe renal or hepatic impairment) at the discretion of the investigator; current evidence, or history of viral hepatitis B or C, acquired immune deficiency syndrome (AIDS) or serological evidence of human immunodeficiency virus HIV, or currently unstable or uncompensated respiratory or cardiac conditions
m) History of bleeding diathesis or long term anticoagulent therapy (other than antiplatelet therapy)
n) Any other reason (e.g., confusion, infirmity, alcoholism), which could jeopardise compliance with the protocol

### TRIAL DRUG/TREATMENT

### 2.3 Formulation, packaging and storage of fulvestrant

Faslodex® (fulvestrant) supplied as a 5% w/v, oily solution containing 250 mg of fulvestrant at a concentration of 50 mg/ml in a volume of 5 ml.

### 2.4 Drug administration: route, dose and regimen

### 2.4.1 Route

Faslodex is administered by intramuscular injection into the gluteus maximus muscle. The injection must be administered slowly over at least 2 minutes. The site of injection should be alternated each month between the left and right buttock. It is recommended that the injection is given with the patient lying on her side.

### 3 CONCURRENT TREATMENT

Except for the study treatment, no systemic treatment known to have an effect on breast cancer, may be used during the trial (except bisphosphonates and tibolone).

Oestrogen replacement therapy stopped before study treatment begins.

LH-RH analogue therapy stopped at least 3 months before enrolment and biochemical evidence must be in the postmenopausal range.

Other drugs not given as systemic treatment for breast cancer but which can affect sex-hormone status or disease response not to commence during the trial. However, the patient can continue to receive such drugs if they were being taken prior to the start of the trial and the investigator is satisfied that the patient's hormone status is stable.

These include:
ketoconazole (antifungal)
prednisolone
adrenocortical suppressants
low dose progestins (for the treatment of hot flushes)

Radiotherapy may be given concurrently for control of bone pain or for other reasons. However, those lesions irradiated will not be included in assessment of response except as progressive disease. The patient will be regarded as having disease progression when radiotherapy is administered for the appearance of new lesions or for objective progression of existing bone lesions. The patient will not be regarded as having disease progression when radiotherapy is administered for other reasons e.g. control of bone pain without objective progression or appearance of new lesions.

Patients taking bisphosphonate treatment may enter the trial. Their bone lesions will not be evaluable for assessment of response except as progressive disease. Patients with bone only disease who are on bisphosphonate treatment are not eligible for the trial.

If bisphosphonates are commenced during the trial in the absence of objective evidence of progression, bone lesions will no longer be considered evaluable for response, but only for progression. Patients requiring initiation of bisphosphonate treatment during the trial for the development of new or progression of existing bone lesions will be regarded as having progression of disease.

Patients receiving long term anticoagulant therapy (other than antiplatelet therapy) are ineligible for the trial because of the risk of intramuscular hemorrhage following the fulvestrant injection. Patients who need to begin anticoagulant therapy while receiving fulvestrant treatment may continue treatment at the discretion of the Investigator. There is an increased risk of hemorrhage in these patients and the Investigator should decide whether that risk is outweighed by the possible benefits of continued fulvestrant treatment. The appropriate clotting time, as measured by the INR, should be checked to ensure that it is within the appropriate therapeutic range prior to each fulvestrant injection. If the INR is above the upper limit of the recommended range, the injection should be withheld until the INR has returned to the therapeutic range.

### 4 TRIAL METHODS

### 4.1 Procedural Summary

A summary of assessments is shown in the Trial Plan, Table 1. Table 1 provides the schedule of assessments to be performed at each of the patient visits. Day 1 is the day on which the patient first receives her fulvestrant injection (not the day of enrolment). Screening data will be used as baseline measurements, except for hematology and biochemistry results, if they are taken > 2 weeks before day 1. Heart rate and blood pressure must be assessed prior to treatment on day 1. The most recent assessment prior to first dosing should be entered on to the Visit 1 CRF. At analysis, eligibility (and hence possible protocol violations) will be based on the Visit 1 data.

Tumour samples do not need to be stained for HER2 protein overexpression before inclusion of the patient in the study and can therefore be tested after registration of the patient

All patients will be assessed every month for the first 3 months and then at 3 month intervals until evidence of:
objective progression as defined in this protocol, regardless of any change in breast cancer therapy after entering the trial
or: death without objective progression.

Screening assessments should be completed within three weeks before enrolment of the patient and the patient should be treated within one week following enrolment. Therefore, baseline tumour assessment should be done within 4 weeks before treatment (except for isotopic bone scans which should be done within 8 weeks before treatment). Any hotspots identified on the bone scan must be confirmed by X-ray or CT scan or MRI within 4 weeks prior to treatment. Patients will then be assessed every month for the first 3 months of treatment and every 3 months thereafter. These visits should occur every 28 days ± 3 days of the protocol visit times. Tumour assessments should occur within +/- 2 weeks of the specified timepoint. fulvestrant injections should occur at regular intervals of 1 month (28 days ± 3 days) in line with assessment visits.

Efficacy will be assessed by objective tumour assessment every 3 months using the appropriate method. Patients with palpable soft-tissue lesions will be evaluated monthly for the first 3 months, then every 3 months thereafter. Tumour assessments should occur within +/- 2 weeks of the scheduled visit times and should be repeated at cessation of trial therapy. Tumour assessments should continue in all patients until progression of disease occurs.

### 4.2 Clinical and laboratory evaluations

### 4.2.1.1 Concurrent conditions

An assessment will be made at screening and at all subsequent visits of concurrent conditions including cancer-related conditions. Any adverse findings at the subsequent visits will be recorded on the Adverse Event form. Only those findings that are in addition to the breast cancer and concurrent metastatic disease will be recorded. Conditions, which are unequivocally related to disease progression, will not be recorded as an adverse event.

Concomitant therapies for concurrent conditions will be recorded.

### 4.2.2 Hematology and biochemistry

Hematology and biochemistry assessments are to be performed at screening and at day 1. However, if screening samples are performed within two weeks prior day 1, no additional samples are required at day 1. Thereafter, hematology and biochemistry assessments are to be performed at 3 month intervals until withdrawal from trial therapy. Laboratory assessments and INR will be analysed at the centres. The parameters detailed below will be assessed.

### 4.2.2.1 Hematology

Hemoglobin
Hematocrit
Platelet count
White cell count (Total)
Neutrophils

An INR sample should be taken at baseline.

### 4.2.2.2 Serum Biochemistry

Creatinine
Albumin*
Alkaline phosphatase (ALP)
Alanine aminotransferase (ALT)
Calcium

* Only to be assessed in case of high calcium (= upper limit of the reference range)

### 4.2.3 HER-2/neu

Tumour samples do not need to be stained for HER2 protein overexpression before inclusion of the patient in the study and can therefore be tested after registration of the patient

### 4.2.4 Vital signs

Blood pressure and heart rate will be assessed prior to treatment on day 1. Assessment should then be performed at every visit thereafter up to and at the time of withdrawal from trial treatment.

### 4.2.5 Weight

Weight will be assessed at Day 1, at every visit thereafter up to and at the time of withdrawal from trial therapy.

### 4.2.6 Performance status

Performance status will be assessed at screening (within 2 weeks prior to enrolment), at every visit thereafter up to and at the time of withdrawal from trial therapy.

### 4.2.7 Chest X-ray / Chest CT-scan

A chest X-ray or a chest CT-scan will be carried out in all patients within 4 weeks before treatment to assess lung condition at entry. Results from the chest X-ray or the chest CT-scan must be included in the objective disease assessment.

### 4.2.8 Bone scan

An isotopic bone scan will be carried out for all patients within 8 weeks before treatment to assess bone disease at entry. An isotopic bone scan is only required at baseline.

Bone lesions indicated in the bone scan which are to be used as baseline evaluable lesions must be confirmed by X-ray or CT-scan or MRI within 4 weeks prior to before treatment.

### 4.2.9 Objective tumour assessment

Baseline objective tumour assessment must be performed no more than 4 weeks prior to treatment except for isotopic bone scan which must be performed no more than 8 weeks before treatment. Any hotspots identified on the bone scan must be confirmed by X-ray or CT scan or MRI within 4 weeks prior to treatment. At entry to the trial, lesions at all sites of disease should be identified as measurable, or evaluable but non-measurable, or neither measurable nor evaluable.

### Patients must have at least one measurable or evaluable lesion to be eligible for the trial.

All clearly defined measurable and evaluable lesions will need to be identified and followed throughout the trial period. The 4 largest and clearly measurable lesions and 4 evaluable lesions will be recorded on the appropriate CRF. Any additional measurable or evaluable lesions will be followed as well as additional lesions. For all evaluable lesions, measurements are not required but radiographs, scans or photographs must be available to support Investigator assessments.

Objective tumour assessment for all patients will be performed within 4 weeks prior to treatment (baseline), and will then be repeated every 3 months (± 2 weeks) after the day of the first fulvestrant injection until objective disease progression. Patients with soft-tissue lesions will also be assessed every month for the first 3 months. Follow-up assessments of evaluable bone lesions must be by X-ray or CT scan or MRI, not isotopic bone scan.

Patients who cease fulvestrant treatment for reasons other than disease progression must still be monitored for objective tumour assessments every 3 months, irrespective of any subsequent change in treatment. Every attempt must be made to maintain the schedule of assessments as indicated in order to avoid possible bias in the estimation of time to disease progression between the treatment groups. Assessments may be performed before scheduled times only if disease progression is suspected.

For each selected measurable lesion, 2 dimensions must be recorded (length and width), axes must be perpendicular and in the same plane. For each evaluable lesion one of the following responses should be assigned by the investigator: complete response (CR), partial response (PR), stable disease (SD) or progression. If more than 4 measurable or 4 evaluable lesions are present at baseline, then these should be followed as additional lesions and an investigator assignment of CR, PR, SD or progression is required at each visit.

The overall response to treatment will be determined according to WHO guidelines

It is recommended that:
- hepatic or pelvic lesions be assessed using abdominal and pelvic computed tomograph (CT) scans. Ultrasound scans of hepatic lesions are acceptable provided that the examination is performed by the same radiologist on each occasion and photographic records of representative lesions are kept. Radioisotope liver scans are not acceptable;
- pulmonary lesions be assessed using chest X-ray or chest CT scan;
- osteolytic bone lesions be assessed using X-ray or CT scan or MRI.

(Bone lesions are not measurable. Osteolytic bone lesions are evaluable but non-measurable; osteoblastic and osteosclerotic bone lesions are neither measurable nor evaluable and therefore not eligible for assessment).

### ADVERSE EVENTS and PATIENT WITHDRAWALS

### 4.3 Adverse events

### 4.3.1 Definition of adverse events

An adverse event (AE) is defined as the development of a new, undesirable medical condition or the deterioration of a pre-existing medical condition following or during exposure to a pharmaceutical product. This definition includes events occurring during any run-in or washout periods, and any follow-up period specified in the trial protocol. The medical condition does not necessarily have a causal relationship with exposure to the pharmaceutical product. A medical condition can be symptoms (such as nausea, chest pain), signs (such as tachycardia, enlarged liver) or abnormal results on investigation (including blood tests, X-rays or scans of various types or electrocardiogram).

For the purpose of this trial, any detrimental change in a patient's condition subsequent to them entering the trial and during the 8 weeks after the last fulvestrant injection, which is not unequivocally due to progression of disease, should be considered to be an adverse event. When there is a deterioration in the condition for which the medicine is being used, there may be uncertainty as to whether this is due to lack of efficacy or an adverse event. In such cases, unless AstraZeneca or the reporting physician consider that the medicine contributed to the deterioration, or local regulations state the contrary, it should be considered to be a lack of efficacy. The development of a new cancer should be regarded as an adverse event. New cancers are those that are not the primary reason for the administration of the trial treatment and have been identified after inclusion into the clinical trial.

### PATHOLOGY

### ASSESSMENT OF HER-2 OVEREXPRESSION

### PATHOLOGY

### Assessment of HER-2 overexpression

### Testing methods

The HER-2 protein is overexpressed in 20-30% of human breast carcinomas as a result of gene amplication and/or transcriptional alterations. Several techniques have been described to detect HER-2 alterations. DNA-based techniques (such as FISH and PCR) have been used to determine gene copy numbers. However, these techniques are not broadly available in every pathology institute. Immunohistochemistry using antibodies directed against the protein have been also wildly used. There is a high level of correlation between FISH and immunohistochemistry in the evaluation in HER-2 status of breast cancers using formalin fixed paraffin-embedded specimen (Jacobs T W, 1999). The advantage of immunohistochemical analysis is the availability of this technique in every pathology institute. Slides from paraffin blocks show reproducible results after even 5-10 years. Therefore the level of HER-2 overexpression can be determined at the time of metastatic disease either on slides of the primary tumor or on the actual metastases.

The HercepTest™ (FDA approved) from DAKO Diagnosis AG was chosen to determine the HER-2 protein overexpression by immunohistochemistry because of its controlled and standardized immunohistochemical staining, its provided control slides and its standardized readout. DAKO HercepTest™ uses an anti-HER-2 polyclonal antibody (A0485).

In the present study, the determination of HER2 status will not influence treatment decisions, but will be of valuable help for information on prognosis and response to fulvestrant.

### WHO PERFORMANCE STATUS

| | Score |
|---|---|
| Fully active, able to carry out all usual activities without restrictions and without the aid of analgesia. | 0 |
| Restricted in strenuous activity, but ambulatory and able to carry out light work or pursue a sedentary occupation. This group also contains patients who are fully active, as in Grade 0, but only with the aid of analgesics. | 1 |
| Ambulatory and capable of all self-care, but unable to work. Up and about more than 50% of waking hours. | 2 |
| Capable of only limited self-care, confined to bed or chair more than 50% of waking hours. | 3 |
| Completely disabled, unable to carry out any self-care and confined totally to bed or chair. | 4 |

### DEFINITION OF MEASURABLE AND EVALUABLE LESIONS AND OBJECTIVE RESPONSE CRITERIA (UICC) AND DETERMINATION OF OVERALL RESPONSE IN SOLID TUMORS (WHO)

### 1. Definition of measurable and evaluable lesions

### (a) Neither measurable nor evaluable

- lesions in previously irradiated fields, unless there is definitive progression at such sites immediately before entry and more than 4 weeks has elapsed between radiotherapy and entry
- lymphoedema
- hilar enlargement
- pleural effusion
- ascites
- metastases in the Central Nervous System
- bone marrow infiltration
- osteoblastic bone lesions
- Osteolytic bone lesions if subject taking bisphosphonates

### (b) Measurable

- lesions that are clinically measurable in two perpendicular axes with at least one dimension
   being ≥2.5 cm e.g. skin nodules or superficial lymph nodes which clinically are clearly involved
- lesions that are measurable using imaging in two perpendicular axes with both dimensions being ≥1.0 cm e.g. nodular lung metastases surrounded by aerated lung, deeply located lesions on ultrasound, CT scan or magnetic resonance imaging (MRI). (Up to 4 measurable lesions [largest and most clearly defined] will be recorded)

### (c) Evaluable but non-measurable

- lesions that can be measured in two perpendicular axes but with one dimension <1.0 cm (measured by imaging) or both dimensions <2.5 cm at baseline (clinically measured)
- lesions that can be measured in one axis only
- visible but non-measurable lesions that may be photographed (e.g. skin infiltration)
- radiographically assessable lesions, e.g. mediastinal lymph nodes, diffuse pulmonary infiltration
- osteolytic bone lesions assessed by plain radiography, CT scanning or MRI.

(Up to 4 [most representative] non-measurable but evaluable lesions will be recorded).

### Patients with bone-only disease taking bisphosphonates must not be entered.

### 2. Objective response criteria

### (a) Complete response

No clinical, radiological or biochemical evidence of residual lesions determined on assessment, provided that there is no evidence of disease progression within 4 weeks of the response and the patient has not died. In the case of lytic bone metastases these must be shown radiologically to have calcified.

CR in patients with "bone only" disease
- all lytic lesions must have remineralised;
- no bone pain should be present (without analgesic);
- no pathological fractures should occur within 4 weeks before and 4 weeks after the assessment;
- remodelling of previous distorted skeletal structure must be evident;
- no new bone lesions;
- bone scan must have normalised, i.e. all 'hot spots' have resolved.

### (b) Partial Response

If there is no evidence of disease progression (i.e., no progression of any lesion and no new lesions) and the patient has not died within 4 weeks of the response then any of the following changes in the objective assessments (compared with entry) will be sufficient evidence of a partial objective response:

For measurable lesions, a decrease of at least 50% compared with entry, in the sum of the products of the two largest perpendicular diameters of all the measurable lesions, without an increase of more than 25% in the size of any lesion or the appearance of any new lesion;

For evaluable, non-measurable lesions, estimated objective improvement of evaluable lesions of 50% or more, observations to be based on radiological, ultrasound or photographic evidence.

PR in patients with bone only disease: partial decrease in size and partial remineralisation of lytic lesions, no pathological fracture and no new bone lesions.

### (c) Disease progression

Any of the following will be sufficient evidence for disease progression:
- increase in the size (product) of more than 25% of any lesion compared with the minimum dimensions recorded during the trial;
- appearance of any new lesion or worsening of any existing evaluable lesion, observations to be based upon radiological, ultrasound or photographic evidence.

### (d) Stable disease

Lack of objective disease progression and insufficient evidence for complete or partial objective response will be classified as stable disease.

### 3. Determination of overall response in solid tumors

The overall response will be determined as described in the WHO Handbook for reporting results of cancer treatment, WHO Offset Publication No. 48, Geneva, 1979, 1985:
- If both measurable and unmeasurable disease is present in a given patient, the result of each should be recorded separately. Note that an overall assessment of response involves parameters.
- In patients with measurable disease, the poorest response designation shall prevail.
- "No change" in unmeasurable lesions will not detract from a partial response in measurable lesion but will reduce a complexe response in measurable lesions to partial response overall.
- If in the totals of responses by organ site there are equal or greater numbers of complete plus partial responses than of "no change" designations, then the overall response will be partial.
- If progressive disease exists in any lesion or when a new lesion appears, then the overall result will be "progressive disease".

## Claims

1. Use of fulvestrant in the preparation of a medicament for the treatment of a patient with breast cancer who previously has been treated with an aromatase inhibitor and tamoxifen and has failed with such previous treatment.

2. A use as claimed in claim 1 provided that the patient has not been prior treated for breast cancer with more than 2 different hormonal agents.

3. A use as claimed in claim 1 or 2 wherein the treatment with tamoxifen preceded the treatment with the aromatase inhibitor.

4. A use as claimed in any claim from 1 to 3 wherein the aromatase inhibitor is selected from anastrazole, letrozole and aminogluethimide.

5. A use as claimed in any claim from 1 to 4 wherein the aromatase inhibitor is anastrazole.

6. A use as claimed in any claim from 1 to 5 wherein fulvestrant is to be administered by intramuscular injection.

## Patentansprüche

1. Verwendung von Fulvestrant bei der Herstellung eines Arzneimittels zur Behandlung einer Brustkrebspatientin, bei der die vorangegangene Behandlung mit einem Aromataseinhibitor und Tamoxifen fehlschlug.

2. Verwendung nach Anspruch 1, mit der Maßgabe, dass die Patientin zuvor nicht mit mehr als 2 unterschiedlichen Hormonmitteln gegen Brustkrebs behandelt wurde.

3. Verwendung nach Anspruch 1 oder 2, wobei die Behandlung mit Tamoxifen vor der Behandlung mit dem Aromataseinhibitor durchgeführt wurde.

4. Verwendung nach einem der Ansprüche 1 bis 3, wobei der Aromataseinhibitor unter Anastrazol, Letrozol und Aminoglutethimid ausgewählt ist.

5. Verwendung nach einem der Ansprüche 1 bis 4, wobei es sich bei dem Aromataseinhibitor um Anastrazol handelt.

6. Verwendung nach einem der Ansprüche 1 bis 5, wobei Fulvestrant als intramuskuläre Injektion zu verabreichen ist.

## Revendications

1. Utilisation de fulvestrant dans la préparation d'un médicament destiné au traitement d'une patiente ayant un cancer du sein qui a été traitée au préalable avec un inhibiteur de l'aromatase et avec du tamoxifène et pour qui un tel traitement préalable a échoué.

2. Utilisation selon la revendication 1, à condition que la patiente n'ait pas été traitée auparavant pour un cancer du sein avec plus de deux agents hormonaux.

3. Utilisation selon la revendication 1 ou 2, dans laquelle le traitement avec le tamoxifène a précédé le traitement avec l'inhibiteur de l'aromatase.

4. Utilisation selon l'une quelconque des revendications 1 à 3, dans laquelle l'inhibiteur de l'aromatase est choisi parmi l'anastrazole, le létrozole et l'aminoglutéthimide.

5. Utilisation selon l'une quelconque des revendications 1 à 4, dans laquelle l'inhibiteur de l'aromatase est l'anastrazole.

6. Utilisation selon l'une quelconque des revendications 1 à 5, dans laquelle le fulvestrant est à administrer par une injection intramusculaire.
